(19) Europäisches Patentamt
European Patent Office
Office européen des brevets

(11) EP 0 940 149 A1

(12) **EUROPEAN PATENT APPLICATION**

(43) Date of publication:
**08.09.1999 Bulletin 1999/36**

(51) Int. Cl.[6]: **A61L 15/60**, B01J 20/26, C08K 5/109

(21) Application number: **99104212.8**

(22) Date of filing: **02.03.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **04.03.1998 JP 5158498**

(71) Applicant:
**Nippon Shokubai Co., Ltd.**
**Osaka-shi, Osaka 541-0043 (JP)**

(72) Inventors:
- **Nagasuna, Kinya**
  **Himeji-shi, Hyogo 671-1242 (JP)**
- **Shimomura, Tadao**
  **Toyonaka-shi, Osaka 565-0083 (JP)**

(74) Representative:
**Henkel, Feiler, Hänzel**
**Möhlstrasse 37**
**81675 München (DE)**

(54) **"Water-absorbing agent and production process therefor"**

(57) The present invention provides a process for producing a water-absorbing agent by using a crosslinking agent with a specific structure, wherein the water-absorbing agent is excellent with good balance in respect to the absorption capacities under no load and under a load and can display excellent absorption properties even if the weight percentage of the water-absorbent resin (resin concentration) is high when the water-absorbent resin is used for materials such as sanitary materials. A water-absorbent resin is mixed with a crosslinking agent, and the resultant mixture is treated, wherein the crosslinking agent has a plurality of structural units of general formula (1) below:

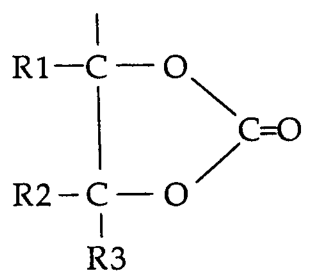

(1)

wherein each of R1~R3 denotes a hydrogen atom, an alkyl group, an aromatic group, a halogen group, a substituted alkyl group, or a substituted aromatic group.

EP 0 940 149 A1

## Description

## BACKGROUND OF THE INVENTION

### A. TECHNICAL FIELD

The present invention relates to a production process for a water-absorbing agent which is favorably used for sanitary materials such as paper-made diapers (disposable diapers), sanitary napkins and so-called incontinent pads.

### B. BACKGROUND ART

In recent years, water-absorbent resins that are hydrophilic resins are widely used as constituent materials of sanitary materials, such as disposable diapers, sanitary napkins, and so-called incontinent pads, for the purpose of causing the water-absorbent resins to absorb body fluids.

Known examples of the above water-absorbent resins are as follows: crosslinked polymers of partially neutralized polyacrylic acids; hydrolyzed products of starch-acrylic acid graft polymers; saponified products of vinyl acetate-acrylic acid ester copolymers; hydrolyzed products of acrylonitrile-or acrylamide copolymers, and their crosslinked polymers; and crosslinked polymers of cationic monomers.

Examples of the properties which the above water-absorbent resins should have are as follows: upon contact with aqueous liquids such as body fluids, excellent water absorption amount or speed, the liquid permeability, the gel strength of the swollen gel, the suction power to suck up water from a base material containing aqueous liquids. However, relations between these properties do not necessarily display positive correlations. For example, as the absorption capacity under no load increases, the absorption properties under a load deteriorate.

As to a method for improving such water-absorption properties of the water-absorbent resin in good balance, there is a known art in which the neighborhood of the surface of the water-absorbent resin is crosslinked, and various methods have been proposed as such.

For example, there are known methods in which the following materials are used as the crosslinking agents: polyhydric alcohols (JP-A-58-180233 and JP-A-61-016903); polyglycidyl compounds, polyaziridine compounds, polyamine compounds, or polyisocyanate compounds (JP-A-59-189103); glyoxal (JP-A-52-117393); polyvalent metals (JP-A-51-136588, JP-A-61-257235 and JP-A-62-007745); silane coupling agents (JP-A-61-211305, JP-A-61-252212, and JP-A-61-264006); alkylene carbonates (DE 4020780). In addition, there are also known methods in which the following materials are allowed to be present as third substances for the purpose of improving the dispersibility of the crosslinking agent when the crosslinking agent is mixed or when the crosslinking reaction is carried out: inert inorganic powders (JP-A-60-163956 and JP-A-60-255814); dihydric alcohols (JP-A-01-292004); water along with ether compounds (JP-A-02-153903); alkylene oxide adducts of monohydric alcohols, or organic acid salts, or lactams (EP 555692); and phosphoric acid (Publication of Internal Patent Application as entered the national phase in Japan (Kohyo) No. 08-508517).

However, there are cases where conventional arts of crosslinking the neighborhood of the surface of the water-absorbent resin cannot sufficiently meet the abilities as demanded to the water-absorbent resin to a high degree in recent years. For example, in recent years, the sanitary materials have a tendency to be designed to be thinned, so the water-absorbent resin concentration in the absorbent structure becomes high. Thus, excellent absorption capacities under no load and under a heavy load with good balance are exemplified as the properties that are desired to the water-absorbent resin in the absorbent structure which contains the water-absorbent resin in a large quantity, namely, high concentration, but the above conventional methods are still insufficient to further raise each of the above values.

Furthermore, there is a problem of the safety of the crosslinking agent as used. Generally, when the crosslinking agent has high reactive groups such as epoxy group, the crosslinking agent itself has property to stimulate skin. Thus, not only considering problems on environment of working, but also considering the application to sanitary materials, it is necessary to strictly control factors such as the amount of the crosslinking agent remaining in the resin, and further, complicated operations in the process are necessary for also decreasing the amount of the residual crosslinking agent. In addition, when the crosslinking agent is polyhydric alcohol or the like, the crosslinking agent itself has relatively high safety, but its reactivity is generally so low that its reaction needs high temperature that might deteriorate the water-absorbent resin or lower the properties of the water-absorbent resin during the crosslinking reaction. Therefore, in fact, there has never been an art (for obtaining water-absorbent resins as preferably used for sanitary materials) which is satisfactory with regard to the performance, the safety, and the process.

## SUMMARY OF THE INVENTION

### A. OBJECTS OF THE INVENTION

The present invention was made considering the above problems on the prior arts, and it is an object of the present invention to provide a production process for a water-absorbing agent which is excellent with good balance in respect to the absorption capacities under no load and under a load and can display excellent absorption properties even if the weight percentage of the water-absorbent resin (resin concentration) is high when the water-absorbent resin is used for materials such as sanitary materials. In addition, it is another object of the present invention to provide a process for producing the above water-absorbing agent (which is excellent in the abilities such as absorption capacities under no load and under a load) by using a crosslinking agent with a specific structure. In addition, it is yet another object of the present invention to provide an absorbent article comprising a water-absorbing agent as obtained using a crosslinking agent with a specific structure. In addition, it is yet another object of the present invention to provide a new crosslinking agent which is particularly fit to crosslink water-absorbent resins.

### B. DISCLOSURE OF THE INVENTION

The present inventors studied and studied with encouragement to themselves and great efforts to solve the above problems. As a result, the inventors completed the present invention by finding that the use of a crosslinking agent with a specific structural unit can achieve the absorption properties excellent with good balance in respect to the absorption capacities under no load and under a load.

Thus, a production process for a water-absorbing agent, according to the present invention, comprises the steps of mixing a water-absorbent resin with a crosslinking agent and treating the resultant mixture;

and this process is characterized in that the crosslinking agent has a plurality of (this wording "a plurality of" means "two or more" herein) structural units of general formula (1) below:

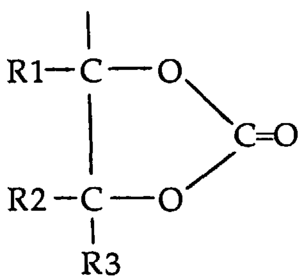

(1)

wherein each of R1~R3 denotes a hydrogen atom, an alkyl group, an aromatic group, a halogen group, a substituted alkyl group, or a substituted aromatic group.

Another production process for a water-absorbing agent, according to the present invention, comprises the steps of mixing a water-absorbent resin with a crosslinking agent and treating the resultant mixture;

and this process is characterized in that the crosslinking agent has one structural unit of general formula (1) above and at least one reactive functional group other than the structural unit of general formula (1) (but not including the case where this at least one reactive functional group is only one hydroxyl group).

A crosslinking agent for water-absorbent resins, according to the present invention, is characterized by having a plurality of structural units of general formula (1) above.

An absorbent article, according to the present invention, is characterized by comprising a water-absorbing agent which is crosslinked with a crosslinking agent having a plurality of structural units of general formula (1) above.

Yet another production process for a water-absorbing agent, according to the present invention, comprises the steps of mixing a water-absorbent resin with a crosslinking agent and treating the resultant mixture;

and this process is characterized in that the crosslinking agent has a plurality of heterocyclic carbonate structural units.

These and other objects and the advantages of the present invention will be more fully apparent from the following detailed disclosure.

## DETAILED DESCRIPTION OF THE INVENTION

Hereinafter, the present invention is explained in detail.

The water-absorbent resin, which is used as the hydrophilic resin to produce the water-absorbing agent of the present invention, is a conventionally known resin that absorbs as large a quantity of water as 50~1,000 times the original in deionized water to thereby form a hydrogel. Particularly, those which have a carboxylic group are preferable and typically obtained by polymerizing and crosslinking hydrophilic monomers of which the main component is either one or both of acrylic acid and a salt (neutralized product) thereof. In addition, as to the above water-absorbent resin, those which have an uncrosslinked water-soluble content of 25 weight % or below, preferably 15 weight % or below, more preferably 10 weight % or below, are used.

Examples of the above salt of acrylic acid include: alkaline metal salts, ammonium salts, and amine salts of acrylic acid. It is preferable that the constituent units of the above water-absorbent resin comprise acrylic acid of 10~40 mol % and its salt of 90~60 mol % (wherein the total of both is 100 mol %). The monomers, as used to produce the water-absorbent resin by polymerizing hydrophilic monomers (of which the main component is either one or both of acrylic acid and a salt thereof), may, if necessary, comprise not only acrylic acid or a salt thereof, but also monomers other than acrylic acid.

The monomers other than acrylic acid are not especially limited, but specified examples of them include the following: anionic unsaturated monomers, such as methacrylic acid, maleic acid, vinylsulfonic acid, styrenesulfonic acid, 2-(meth)acrylamido-2-methylpropanesulfonic acid, 2-(meth)acryloylethanesulfonic acid, and 2-(meth)acryloylpropanesulfonic acid, and their salts; nonionic unsaturated monomers containing a hydrophilic group, such as acrylamide, methacrylamide, N-ethyl(meth)acrylamide, N-n-propyl(meth)acrylamide, N-isopropyl(meth)acrylamide, N,N-dimethyl(meth)acrylamide, 2-hydroxyethyl (meth)acrylate, 2-hydroxypropyl (meth)acrylate, methoxypolyethylene glycol (meth)acrylate, polyethylene glycol mono(meth)acrylate, vinylpyridine, N-vinylpyrrolidone, N-acryloylpiperidine, and N-acryloylpyrrolidine; cationic unsaturated monomers such as N,N-dimethylaminoethyl (meth)acrylate, N,N-diethylaminoethyl (meth)acrylate, N,N-dimethylaminopropyl (meth)acrylate, N,N-dimethylaminopropyl(meth)acrylamide, and their quaternary salts. These monomers may be used either alone respectively or in combinations with each other.

In the present invention, when the monomers other than acrylic acid are used, the ratio of them is preferably 30 mol % or below, more preferably 10 mol % or below, of the total with acrylic acid and its salt. If the above monomers other than acrylic acid are used in the above ratio, then the water-absorption properties of the resultant water-absorbent resin are still more improved, and the water-absorbent resin is obtainable at a still lower cost.

When the above hydrophilic monomer (of which the main component is acrylic acid or a salt thereof) is, for example, polymerized to obtain the water-absorbent resin as used in the present invention, bulk polymerization or precipitation polymerization can be carried out. However, considering the performance or the easiness of the polymerization control, it is preferable to carry out aqueous solution polymerization or reversed-phase suspension polymerization using the above hydrophilic monomer in the form of its aqueous solution. Incidentally, when the monomer is used in the form of its aqueous solution, the concentration of the monomer in its aqueous solution (hereinafter referred to as "aqueous monomer solution") is not especially limited, but is preferably in the range of 10~70 weight %, more preferably 20~40 weight %. In addition, when the above aqueous solution polymerization or reversed-phase suspension polymerization is carried out, a solvent other than water may be jointly used if necessary, and the kind of the solvent as jointly used is not especially limited.

When the above polymerization is initiated, the following radical polymerization initiators, for example, can be used: potassium persulfate, ammonium persulfate, sodium persulfate, t-butyl hydroperoxide, hydrogen peroxide, and 2,2'-azobis(2-aminodipropane) dihydrochloride.

Furthermore, a redox initiator is also available by further using a reductant to promote decomposition of the above polymerization initiator and combining both with each other. Examples of the above reductant include the following: (bi)sulfurous acid salts such as sodium sulfite and sodium hydrogensulfite; L-ascorbic acid (or its salts); reducible metals (or their salts) such as ferrous salts; and amines. However, the reductant is not especially limited to them.

The amount of the above polymerization initiator as used is usually in the range of 0.001~2 mol %, preferably 0.01~0.1 mol %. In the case where the amount of the polymerization initiator is less than 0.001 mol %, there are disadvantages in that a large amount of monomers remain unreacted, so the amount of monomers, remaining in the resultant water-absorbent resin, increases. On the other hand, in the case where the amount of the polymerization initiator exceeds 2 mol %, there might be disadvantages in that the water-soluble content in the resultant water-absorbent resin increases.

In addition, the polymerization reaction may be initiated by irradiating the reaction system with active energy rays, such as radiations, electron beam, and ultraviolet rays.

Incidentally, the reaction temperature in the above polymerization reaction is not especially limited, but is preferably in the range of 20~90 °C. In addition, the reaction time is not especially limited either and may fitly be set according to factors such as the respective kinds of the hydrophilic monomers and polymerization initiators and the reaction temperature.

The water-absorbent resin, used in the present invention, may be a self-crosslinking type using no crosslinking agent, but preferable ones are those which are copolymerized or reacted with an internal-crosslinking agent having two

or more polymerizable unsaturated groups or two or more reactive groups per molecule.

Specified examples of the above internal-crosslinking agent include the following: N,N-methylenebis(meth)acrylamide, (poly)ethylene glycol (meth)acrylate, (poly)propylene glycol di(meth)acrylate, trimethylolpropane tri(meth)acrylate, glycerol tri(meth)acrylate, glycerol acrylate methacrylate, ethylene-oxide-denatured trimethylolpropane tri(meth)acrylate, pentaerythritol hexa(meth)acrylate, triallyl cyanurate, triallyl isocyanurate, triallyl phosphate, triallylamine, poly(meth)allyloxyalkanes, (poly)ethylene glycol diglycidyl ether, glycerol diglycidyl ether, ethylene glycol, polyethylene glycol, propylene glycol, glycerol, pentaerythritol, ethylenediamine, ethylene carbonate, propylene carbonate, polyethylenimine, and glycidyl (meth)acrylate.

These internal-crosslinking agents may be used either alone respectively or in combinations with each other. In addition, these internal-crosslinking agents may be added to the reaction system either all at once or divisionally. When two or more kinds of internal-crosslinking agents are used, it is preferable to essentially use a compound with two or more polymerizable unsaturated groups, considering the absorption properties of the resultant water-absorbent resin.

The amount of the above internal-crosslinking agent as used is preferably in the range of 0.005~2 mol %, more preferably 0.01~1 mol %, of the above hydrophilic monomers. In the respective cases where the amount of the internal-crosslinking agent is smaller than 0.005 mol % and where the amount of the internal-crosslinking agent exceeds 2 mol %, the water-absorbent resin having the desired water absorption properties might not be obtained.

When the crosslinking structure is introduced into the internal portion of the water-absorbent resin using the above internal-crosslinking agent, the internal-crosslinking agent may be added to the reaction system during or after polymerization, or after polymerization and neutralization, of the above hydrophilic monomers.

Incidentally, in the above polymerization, the following materials may be added to the reaction system: various foaming agents such as carbonates (or hydrogencarbonates), carbon dioxide, azo compounds, and inert organic solvents; hydrophilic polymers such as starch-cellulose, derivatives thereof, polyvinyl alcohol, polyacrylic acid (or its salts), and crosslinked polymers of polyacrylic acid (or its salts); various surface-active agents; and chain transfer agents such as hypophosphorous acid (or its salts).

When the water-absorbent resin as obtained by the above polymerization reaction is a gel, the above water-absorbent resin is usually dried and, if necessary, pulverized.

The water content of the water-absorbent resin, usable in the present invention, is not especially limited, but it in the range of usually about 1 ~ about 400 %, preferably 1~40 %, more preferably 1~10 %.

In addition, the particle diameter of the water-absorbent resin, usable in the present invention, may exceed 1,000 μm in terms of average particle diameter of gels that is obtained by the polymerization reaction and has not been dried or pulverized yet. However, the particle diameter is usually in the range of 10~1,000 μm, preferably 50~800 μm, more preferably 75~600 μm, particularly preferably 150~500 μm, on the average. The particle shape of the water-absorbent resin as obtained in this way, for example, may be spherical, pulverized, or irregular, and is not especially limited, but those which have the irregular pulverized shapes, as obtained via the pulverization step, are preferably used.

As to the water-absorbent resin as obtained by the above method, it is preferable to use those which display a high absorption capacity value of 40 g/g or more, preferably 45 g/g or more, under no load, because the effects of the present invention are remarkably shown by such a resin. Of course, the above absorption capacity is fitly adjusted according to the purpose.

The present invention can be achieved by mixing a water-absorbent resin with a crosslinking agent and heating the resultant mixture, wherein the water-absorbent resin is obtainable by the above polymerization, and the crosslinking agent has a specific structure.

A crosslinking agent, usable in the present invention, has a plurality of structural units of general formula (1) above.

Another crosslinking agent, usable in the present invention, has one structural unit of general formula (1) above and at least one reactive functional group other than the structural unit of general formula (1) (but not including the case where this at least one reactive functional group is only one hydroxyl group).

Yet another crosslinking agent, usable in the present invention, has a plurality of heterocyclic carbonate structural units.

The use of these crosslinking agents with the specific structural units can give a water-absorbing agent which is excellent with good balance in respect to the absorption capacities under no load and under a load and can display excellent absorption properties even if the weight percentage of the water-absorbent resin (resin concentration) is high when the water-absorbent resin is used for materials such as sanitary materials.

The crosslinking agent usable in the present invention, having a plurality of structural units of general formula (1) or heterocyclic carbonate structural units, are generally, for example, obtainable by reacting substances such as phosgene or dialkyl carbonate upon compounds having at least two pairs of adjacent hydroxyl groups, or by reacting carbonic acid gas upon polyepoxy compounds under conditions such as high temperature and high pressure. In addition, the crosslinking agent may be obtained by polymerizing monomers having a polymerizable unsaturated group and

a structural unit of general formula (1). In a preferable method, carbonic acid gas is reacted upon polyepoxy compounds.

Examples of the polyepoxy compound, preferably usable in the above case, include: ethylene glycol diglycidyl ether, diethylene glycol diglycidyl ether, polyethylene glycol diglycidyl ether, glycerol polyglycidyl ether, diglycerol polyglycidyl ether, polyglycerol polyglycidyl ether, propylene glycol diglycidyl ether, polypropylene glycol diglycidyl ether, polytetramethylene glycol diglycidyl ether, sorbitol polyglycidyl ether, sorbitan polyglycidyl ether, pentaerythritol polyglycidyl ether, trimethylolpropane polyglycidyl ether, neopentyl glycol diglycidyl ether, triglycidyl tris(2-hydroxyethyl) isocyanurate, and adipic acid diglycidyl ester.

The crosslinking agent (which has one structural unit of general formula (1) and at least one reactive functional group other than the structural unit of general formula (1) (but not including the case where this at least one reactive functional group is only one hydroxyl group)) is, for example, obtainable by reacting substances such as phosgene or dialkyl carbonate upon compounds having one pair of adjacent hydroxyl groups and at least one reactive functional group other than the structural unit of general formula (1) (but not including the case where this at least one reactive functional group is only one hydroxyl group), or by reacting carbonic acid gas upon monoepoxy compounds having at least one reactive functional group other than the structural unit of general formula (1) (but not including the case where this at least one reactive functional group is only one hydroxyl group), such as glycidyl acrylate, glycidyl methacrylate, and allyl glycidyl ether. Examples of the reactive functional group other than the structural unit of general formula (1) include: epoxy group, amino group, isocyanate group, aziridine group, oxazoline group, and polymerizable unsaturated group. Incidentally, when the reactive functional group other than the structural unit of general formula (1) is a hydroxyl group, what has a plurality of hydroxyl groups can preferably be used as the crosslinking agent in the present invention, but what has only one hydroxyl group is unpreferable because its reactivity is low.

A preferable one of the present invention crosslinking agents is what has a plurality of structural units of general formula (1) above and a unit of general formula (2) below:

$$-[C(R4)(R5)-C(R6)(R7)-O]_m- \qquad (2)$$

wherein:

each of R4~R7 denotes a hydrogen atom, an alkyl group, an aromatic group, a halogen group, a substituted alkyl group, or a substituted aromatic group; and
m is an integer of 1 or more.

Another preferable one of the present invention crosslinking agents is what has a plurality of structural units of general formula (1) above and a unit of general formula (3) below:

$$-[C(R8)(R9)-C(OH)(R10)-C(R11)(R12)]_p- \qquad (3$$

wherein:

each of R8~R12 denotes a hydrogen atom, an alkyl group, an aromatic group, a halogen group, a substituted alkyl group, or a substituted aromatic group; and
p is an integer of 1 or more.

In addition, for improving the affinity to the water-absorbent resin and the solubility to water, it is more preferable to use what further has a plurality of hydroxyl groups, still more preferably, what further has a repeating unit skeleton containing a plurality of hydroxyl groups, as the above crosslinking agent preferably usable in the present invention.

In addition, what has a plurality of heterocyclic carbonate structural units is also preferably usable as the crosslinking agent in the present invention. Examples of ring structures in such a crosslinking agent include 5-, 6-, and 7-membered rings. Generally, a hydrogen atom, an alkyl group, an aromatic group, a halogen group, a substituted alkyl group, or a substituted aromatic group is bonded to a carbon atom other than the carbonate moiety of these rings.

The above crosslinking agents of the present invention are preferably usable not only as a surface-crosslinking agent for water-absorbent resins, but also as the above internal-crosslinking agent for water-absorbent resins.

The amount of the crosslinking agent, as used in the present invention, is in the range of about 0.001 ~ about 10 weight parts per 100 weight parts of the water-absorbent resin. Amounts exceeding 10 weight parts are unfavorable, not only because they are uneconomical, but also because they are excessive to the formation of the optimum crosslinking structure in the water-absorbing agent. Furthermore, amounts smaller than 0.001 weight parts make it difficult to obtain effects of improving the properties of the water-absorbing agent such as absorption capacity under a

load.

In the present invention, it is preferable to use water when the water-absorbent resin is mixed with the crosslinking agent. The amount of water, as used, is different according to factors such as the kind, particle diameter, or water content of the water-absorbent resin, but is in the range of preferably 0~20 weight parts (but not including zero), more preferably 0.5~10 weight parts.

In addition, when the water-absorbent resin is mixed with the crosslinking agent or its aqueous solution, hydrophilic organic solvents or third substances may be used as the solvent to raise the miscibility.

When the hydrophilic organic solvent is used, examples thereof include the following: lower alcohols such as methyl alcohol, ethyl alcohol, n-propyl alcohol, isopropyl alcohol, n-butyl alcohol, isobutyl alcohol, and t-butyl alcohol; ketones such as acetone; ethers such as dioxane, tetrahydrofuran, and methoxy(poly)ethylene glycol; amides such as ε-caprolactam and N,N-dimethylformamide; sulfoxides such as dimethyl sulfoxide; polyhydric alcohols such as ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerol, polyglycerol, 2-butene-1,4-diol, 1,3-butanediol, 1,4-butanediol, 1,5-pentanediol, 1,6-hexanediol, 1,2-cyclohexanedimethanol, 1,2-cyclohexanol, trimethylolpropane, diethanolamine, triethanolamine, polyoxypropylene, oxyethylene-oxypropylene block copolymer, pentaerythritol, and sorbitol.

The amount of the hydrophilic organic solvent as used is different according to factors such as the kind, particle diameter, or water content of the water-absorbent resin, but is preferably 20 weight parts or below, more preferably in the range of 0.1~10 weight parts, per 100 weight parts of the solid content of the water-absorbent resin.

In addition, inorganic acids, organic acids, or polyamino acids, as disclosed in EP 0668080, as well as the aforementioned inert inorganic powders may be allowed to coexist as third substances.

A second surface-crosslinking agent may further be used if it does not hinder the effects of the crosslinking agent as used in the present invention. Examples of the second surface-crosslinking agent include the following: polyhydric alcohol compounds such as ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerol, polyglycerol, 2-butene-1,4-diol, 1,3-butanediol, 1,4-butanediol, 1,5-pentanediol, 1,6-hexanediol, 1,2-cyclohexanedimethanol, 1,2-cyclohexanol, trimethylolpropane, diethanolamine, triethanolamine, polyoxypropylene, oxyethylene-oxypropylene block copolymer, pentaerythritol and sorbitol; epoxy compounds such as ethylene glycol diglycidyl ether, polyethylene diglycidyl ether, glycerol polyglycidyl ether, diglycerol polyglycidyl ether, polyglycerol polyglycidyl ether, propylene glycol diglycidyl ether, polypropylene glycol diglycidyl ether and glycidol; polyamine compounds, such as ethylenediamine, diethylenetriamine, triethylenetetraamine, tetraethylenepentaamine, pentaethylenetetraamine and polyethylenimine, and their inorganic or organic salts (for example, azetidinium salts); polyisocyanate compounds such as 2,4-tolylene diisocyanate and hexamethylene diisocyanate; polyoxazoline compounds such as 1,2-ethylenebisoxazoline; alkylene carbonate compounds such as 1,3-dioxolan-2-one, 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one and 1,3-dioxopan-2-one; haloepoxy compounds, such as epichlorohydrin, epibromohydrin and α-methylepichlorohydrin, and their polyamine adducts (for example, Kymene made by Hercules: registered trademark); silane coupling agents such as γ-glycidoxypropyltrimethoxysilane and γ-aminopropyltriethoxysilane; and polyvalent metallic compounds such as hydroxides and chlorides of zinc, calcium, magnesium, aluminum, iron and zirconium.

When the water-absorbent resin is mixed with the crosslinking agent as used in the present invention, it is, for example, permissible to mix the crosslinking agent with a dispersion as prepared by dispersing the water-absorbent resin into the above hydrophilic organic solvent, but the mixing method is not especially limited. In a preferable method among various mixing methods, the crosslinking agent (if necessary, in the form of solution with either one or both of water and the hydrophilic organic solvent) is directly sprayed or dropped to the water-absorbent resin, thereby mixing them. In addition, when water is used for mixing, a water-insoluble fine particle powder or a surface-active agent may be allowed to coexist.

It is preferable that the mixing apparatus, as used to mix the water-absorbent resin and the crosslinking agent in the present invention, has a great mixing force to mix both materials uniformly and surely. Preferable examples of the above mixing apparatus include the following: cylinder type mixers, double-wall cone type mixers, V-character-shaped mixers, ribbon type mixers, screw type mixers, fluidized-furnace rotary disk type mixers, gas current type mixers, double-arm type kneaders, internal mixers, pulverizing type kneaders, rotary mixers, and screw type extruders, and more preferable ones are high-speed agitation type mixers.

The treatment, which is carried out after the water-absorbent resin and the crosslinking agent are mixed, as referred to in the present invention, is a treatment to run the crosslinking reaction, for which it is generally preferable to carry out a heating treatment. The temperature of the above heating treatment is different according to the type of the crosslinking agent as used, but is preferably in the range of 40~250 °C. In the case where the treatment temperature is lower than 40 °C, the uniform crosslinking structure might not be formed, so the water-absorbing agent, excellent in the

balance between the absorption capacities under no load and under a load, might not be obtainable. In the case where the treatment temperature exceeds 250 °C, the deterioration of the water-absorbent resin might occurs to degrade the performance of the water-absorbing agent, so caution is necessary. The crosslinking agent of the present invention is featured by being excellent in the reactivity and by enabling the crosslinking reaction to rapidly run. Therefore, the treatment temperature is more preferably in the range of 70~220 °C, still more preferably in the range of 70~200 °C.

The above heating treatment can be carried out using conventional dryers or heating-furnaces. Examples of the dryers include the following: channel type mixing dryers, rotary dryers, desk dryers, fluidized-bed dryers, gas-stream type dryers, and infrared dryers.

The above-mentioned production process of the present invention can give a water-absorbing agent which is excellent in both absorption capacities under no load and under a load and displays excellent absorption properties even if the weight percentage of the water-absorbent resin (resin concentration) is high when the water-absorbent resin is used for materials such as sanitary materials.

In addition, in the present invention, various functions also can be given to the above water-absorbing agents by further adding thereto the following materials: disinfectants; deodorants; antimicrobial agents; perfumes; various inorganic powders; foaming agents; pigments; dyes; hydrophilic short fibers; manure; oxidants; reductants; water; and salts.

In addition, an absorbent article, according to the present invention, comprises a water-absorbing agent (the above water-absorbing agent of the present invention) which is crosslinked with a crosslinking agent having a plurality of structural units of general formula (1) above.

The above absorbent article of the present invention preferably comprises the absorbent layer which includes the absorbing agent of the above-mentioned constitution and is interposed between the sheet with liquid permeability and the sheet with liquid impermeability. Then, because the absorbent article of the present invention comprises the water-absorbing agent of the present invention, this absorbent article has excellent water absorption properties. Specified examples of the absorbent article include sanitary materials such as paper diapers, sanitary napkins, and so-called incontinence pads, but the absorbent article is not especially limited. Because the absorbent article has excellent water absorption properties, it can prevent urine from leaking and can afford so-called dry feeling in the case where the absorbent article is, for example, a paper diaper.

The above-mentioned sheet with liquid permeability (hereinafter referred to as liquid-permeable sheet) comprises a material that is permeable with aqueous liquids. Examples of the material forming the liquid-permeable sheet include: nonwoven fabrics, woven fabrics; porous synthetic resin films of polyethylene, polypropylene, polyester, polyamide. In addition, the above-mentioned sheet with liquid impermeability (hereinafter referred to as liquid-impermeable sheet) comprises a material that is impermeable with aqueous liquids. Examples of the material forming the liquid-impermeable sheet include: synthetic resin films of polyethylene, polypropylene, ethylene vinyl acetate, polyvinyl chloride; films of combined materials of these synthetic resins with nonwoven fabrics; films of combined materials of the above-mentioned synthetic resins with woven fabrics. Incidentally, the liquid-impermeable sheet may be permeable with vapor.

The constitution of the absorbent layer is not especially limited if it has the above-mentioned water-absorbing agent. In addition, the process for producing the absorbent layer is not especially limited. Furthermore, the process for producing the absorbent article, such as the method for interposing the absorbent layer between the liquid-permeable sheet and the liquid-impermeable sheet, is not especially limited.

(Effects and Advantages of the Invention):

The production process of the present invention can give a water-absorbing agent by a simple process, which agent is excellent with good balance in respect to the absorption capacities under no load and under a load and can display excellent absorption properties even if the weight percentage of the water-absorbent resin (resin concentration) is high when the water-absorbent resin is used for materials such as sanitary materials.

## DETAILED DESCRIPTION OF THE PREFERRED EMBODIMENTS

Hereinafter, the present invention is more specifically illustrated by the following examples of some preferred embodiments in comparison with comparative examples not according to the invention. However, the invention is not limited to these examples. Incidentally, the performances of the water-absorbing agent were measured by the following methods:

(a) Absorption capacity under no load:

First, 0.2 g of water-absorbent resin (water-absorbing agent) was uniformly placed into a nonwoven-fabric-made bag (60 mm × 60 mm) and then immersed into a 0.9 wt % aqueous sodium chloride solution (physiological sodium chloride solution). Sixty minutes later, the bag was drawn up and then drained at 250 G for 3 minutes with a centrifuge, and the weight $W_1$ (g) of the bag was then measured. On the other hand, the same procedure was carried out using no water-absorbing agent, and the resultant weight $W_0$ (g) was measured. Thus, the absorption capacity (g/g) under no load was calculated from these weights $W_1$ and $W_0$ in accordance with the following equation:

$$\text{absorption capacity (g/g) under no load} = (\text{weight } W_1 \text{ (g)} - \text{weight } W_0 \text{ (g)})/(\text{weight (g) of water-absorbing agent}).$$

(b) Absorption capacity under load:

First, 0.9 g of water-absorbent resin (water-absorbing agent) is uniformly spread on a stainless wire net of 400 mesh (mesh size: 38 μm) as attached by fusion to the bottom of a plastic supporting cylinder of inner diameter 60 mm, on which a piston and a load are further mounted in sequence, wherein the piston has an outer diameter only a little smaller than 60 mm and makes no gap with the wall face of the supporting cylinder, but is not hindered from moving up and down, and the total weight of the piston and the load are adjusted to about 565 g to uniformly apply a load of 20 $g/cm^2$ to the water-absorbing agent. Then, the weight (Wa) of the resultant set of measurement apparatus is measured.

A glass filter of 90 mm is mounted inside a Petri dish of 150 mm, and a 0.9 wt % aqueous NaCl solution is added up to the same level as the surface of the glass filter, on which a GF/A filter paper of diameter 9 cm for glass filter is then mounted such that its entire surface will be wetted, and the excessive liquid is removed.

The above set of measurement apparatus is mounted on the above wet filter paper for glass filter, thereby allowing the water-absorbing agent to absorb the liquid under a load. After 1 hour, the set of measurement apparatus is removed by lifting it, and its weight (Wb) is measured again. The absorption capacity under a load is determinable in accordance with the following equation:

$$\text{Absorption capacity under load (g/g)} = (Wb - Wa)/0.9$$

REFERENTIAL EXAMPLE 1

A reaction solution to produce a water-absorbent resin was prepared by dissolving 4.0 weight parts of polyethylene glycol diacrylate (n= 8) (as the internal-crosslinking agent) into 5,500 parts of a 33 weight % aqueous solution of sodium acrylate (neutralization ratio: 75 mol %) (as the monomer component). Next, this reaction solution was degassed under a nitrogen gas atmosphere for 30 minutes.

Then, the reaction solution was supplied into a reaction vessel as prepared by capping a stainless-steel-made double-arm type kneader having two sigma type wings and a jacket. While maintaining the reaction solution at 30 °C, the atmosphere inside the above reaction vessel was replaced with a nitrogen gas. Next, while the reaction solution was stirred, 2.4 parts of ammonium persulfate and 0.12 parts of L-ascorbic acid were added to the reaction solution, so that a polymerization reaction got started about 1 minute after. The polymerization was carried out at 30-80 °C, and the resultant hydrogel polymer was got out 60 minutes after the initiation of the polymerization.

The resultant hydrogel polymer was spread on a wire net of 300 μm in mesh size and dried at 150 °C with hot air for 90 minutes. Then, the resultant dried product was pulverized with a vibration mill and further classified with a wire net of 500 μm in mesh size, thus obtaining water-absorbent resin (1) as pulverized into the irregular shape with an average particle diameter of 400 μm, wherein 0.5 weight % of water-absorbent resin (1) had a particle diameter less than 150 μm.

REFERENTIAL EXAMPLE 2

Water-absorbent resin (2) was obtained in the same way as of Referential Example 1 except that the amount of polyethylene glycol diacrylate (n= 8) was changed to 4.9 weight parts. The resultant water-absorbent resin (2) had an average particle diameter of 380 μm and was the irregular pulverized shape. In addition, 0.7 weight % of water-absorbent resin (2) had a particle diameter less than 150 μm.

EXAMPLE 1

One hundred weight parts of water-absorbent resin (1), as obtained in Referential Example 1, was mixed with a crosslinking agent solution comprising 0.15 weight part of a crosslinking agent, 5 weight parts of water, and 2 weight parts of isopropyl alcohol, wherein the crosslinking agent is obtained by reacting carbonic acid gas upon ethylene glycol diglycidyl ether, namely, by deriving two structural units of general formula (1) (wherein R1~R3 are hydrogen atoms) from two epoxy groups of both terminals of ethylene glycol diglycidyl ether. The resultant mixture was heated at 140 °C

for 60 minutes to obtain water-absorbing agent (1). Water-absorbing agent (1) displayed only a little lower absorption capacity under no load and a much higher absorption capacity under a load than water-absorbent resin (1).

EXAMPLE 2

One hundred weight parts of water-absorbent resin (2), as obtained in Referential Example 2, was mixed with a crosslinking agent solution comprising 0.3 weight part of a crosslinking agent, 5 weight parts of water, and 2 weight parts of isopropyl alcohol, wherein the crosslinking agent is obtained by reacting carbonic acid gas upon ethylene glycol diglycidyl ether, namely, by deriving two structural units of general formula (1) (wherein R1~R3 are hydrogen atoms) from two epoxy groups of both terminals of ethylene glycol diglycidyl ether. The resultant mixture was heated at 190 °C for 60 minutes to obtain water-absorbing agent (2), which displayed an absorption capacity of 33 (g/g) under no load and an absorption capacity of 26 (g/g) under a load.

EXAMPLE 3

One hundred weight parts of water-absorbent resin (2), as obtained in Referential Example 2, was mixed with a crosslinking agent solution comprising 0.5 weight part of a crosslinking agent, 6 weight parts of water, and 3 weight parts of ethyl alcohol, wherein the crosslinking agent is obtained by reacting carbonic acid gas upon diethylene glycol diglycidyl ether, namely, by deriving two structural units of general formula (1) (wherein R1~R3 are hydrogen atoms) from two epoxy groups of both terminals of diethylene glycol diglycidyl ether. The resultant mixture was heated at 200 °C for 60 minutes to obtain water-absorbing agent (3), which displayed an absorption capacity of 32 (g/g) under no load and an absorption capacity of 26 (g/g) under a load.

COMPARATIVE EXAMPLE 1

Water-absorbent resin (2) was regarded as comparative water-absorbing agent (1), and its properties were measured. Comparative water-absorbing agent (1) displayed an absorption capacity of 42 (g/g) under no load and an absorption capacity of 9 (g/g) under a load.

Various details of the invention may be changed without departing from its spirit not its scope. Furthermore, the foregoing description of the preferred embodiments according to the present invention is provided for the purpose of illustration only, and not for the purpose of limiting the invention as defined by the appended claims and their equivalents.

## Claims

1. A production process for a water-absorbing agent, comprising the steps of mixing a water-absorbent resin with a crosslinking agent and treating the resultant mixture;
   with the process being characterized in that the crosslinking agent has a plurality of structural units of general formula (1) below:

   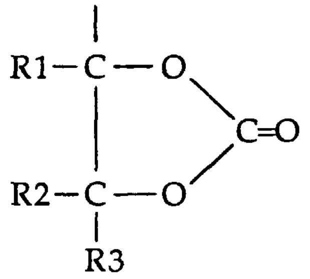

   (1)

   wherein each of R1~R3 denotes a hydrogen atom, an alkyl group, an aromatic group, a halogen group, a substituted alkyl group, or a substituted aromatic group.

2. A production process for a water-absorbing agent, comprising the steps of mixing a water-absorbent resin with a crosslinking agent and treating the resultant mixture;
   with the process being characterized in that the crosslinking agent has one structural unit of general formula (1) below and at least one reactive functional group other than the structural unit of general formula (1) (but not including the case where the at least one reactive functional group is only one hydroxyl group), wherein general formula (1) is:

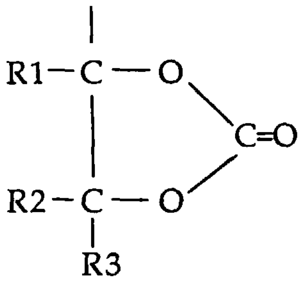

(1)

wherein each of R1~R3 denotes a hydrogen atom, an alkyl group, an aromatic group, a halogen group, a substituted alkyl group, or a substituted aromatic group.

**3.** A production process according to claim 1 or 2, wherein the water-absorbent resin has a carboxyl group.

**4.** A crosslinking agent for water-absorbent resins, characterized by having a plurality of structural units of general formula (1) below:

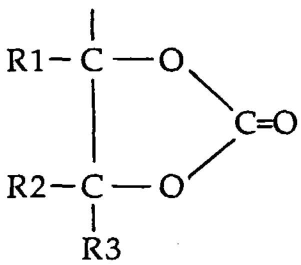

(1)

wherein each of R1~R3 denotes a hydrogen atom, an alkyl group, an aromatic group, a halogen group, a substituted alkyl group, or a substituted aromatic group.

**5.** A crosslinking agent according to claim 4, which further has a unit of general formula (2) below:

$$-[C(R4)(R5)-C(R6)(R7)-O]_m- \quad (2)$$

wherein:

each of R4~R7 denotes a hydrogen atom, an alkyl group, an aromatic group, a halogen group, a substituted alkyl group, or a substituted aromatic group; and
m is an integer of 1 or more.

**6.** A crosslinking agent according to claim 4, which further has a unit of general formula (3) below:

$$-[C(R8)(R9)-C(OH)(R10)-C(R11)(R12)]_p- \quad (3)$$

wherein:

each of R8~R12 denotes a hydrogen atom, an alkyl group, an aromatic group, a halogen group, a substituted alkyl group, or a substituted aromatic group; and
p is an integer of 1 or more.

**7.** A crosslinking agent according to claim 4, which further has a plurality of hydroxyl groups.

8. An absorbent article, comprising a water-absorbing agent which is crosslinked with a crosslinking agent having a plurality of structural units of general formula (1) below:

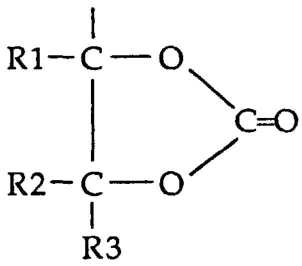

(1)

wherein each of R1~R3 denotes a hydrogen atom, an alkyl group, an aromatic group, a halogen group, a substituted alkyl group, or a substituted aromatic group.

9. A production process for a water-absorbing agent, comprising the steps of mixing a water-absorbent resin with a crosslinking agent and treating the resultant mixture;
with the process being characterized in that the crosslinking agent has a plurality of heterocyclic carbonate structural units.

European Patent Office

# EUROPEAN SEARCH REPORT

Application Number: EP 99104212.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 6) |
|---|---|---|---|
| D,A | <u>DE 4020780 C1</u> (CHEMISCHE FABRIK STOCKHAUSEN GmbH) 29 August 1991 (29.08.91), claims, page 3, lines 34-37. -- | 1-9 | A 61 L 15/60<br>B 01 J 20/26<br>C 08 K 5/109 |
| D,A | <u>EP 0668080 A2</u> (NIPPON SHOKUBAI CO., LTD.) 23 August 1995 (29.08.95), abstract, page 5, lines 45,51, page 8, line 47 - page 9, line 57. -- | 1-9 | |
| A | <u>EP 0712659 A1</u> (NIPPON SHOKUBAI KABUSHIKI KAISHA) 22 May 1996 (22.05.96), claims 21-30, page 7, line 47 - page 8, line 33. -- | 1-9 | |
| A | Patent Abstracts of Japan, Vol. 5, No. 016, 31 May 1996; & JP 08-027372 A (NIPPON SHOKUBAI CO. LTD.) 31 January 1996, abstract. ---- | 1-9 | TECHNICAL FIELDS SEARCHED (Int. Cl. 6)<br>A 61 F<br>A 61 L<br>B 01 J<br>C 08 K |

The present search report has been drawn up for all claims

| Place of search | Date of completion of the search | Examiner |
|---|---|---|
| VIENNA | 26-05-1999 | SCHÄFER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

ANNEX TO THE EUROPEAN SEARCH REPORT
ON EUROPEAN PATENT APPLICATION NO. EP 99104212.8

This annex lists the patent family members relating to the patent documents cited in the above-mentioned search report.
The members are as contained in the EPIDOS INPADOC file on 1. 6.1999.
The European Patent Office is in no way liable for these particulars which are merely given for the purpose of information.

| Patent document cited in search report | | Publication date | Patent family member(s) | | Publication date |
|---|---|---|---|---|---|
| DE C1 | 4020780 | 29-08-1991 | AT E | 140870 | 15-08-1996 |
| | | | AU A1 | 68894/91 | 23-01-1992 |
| | | | AU B2 | 649240 | 19-05-1994 |
| | | | CA AA | 2086002 | 30-12-1991 |
| | | | DE C0 | 59010437 | 05-09-1996 |
| | | | DK T3 | 536128 | 30-12-1996 |
| | | | EP A1 | 536128 | 14-04-1993 |
| | | | EP B1 | 536128 | 31-07-1996 |
| | | | ES T1 | 2053426 | 01-08-1994 |
| | | | ES T3 | 2053426 | 16-11-1996 |
| | | | FI A | 925892 | 28-12-1992 |
| | | | FI A0 | 925892 | 28-12-1992 |
| | | | HU A0 | 9204134 | 28-04-1993 |
| | | | HU A2 | 63345 | 30-08-1993 |
| | | | JP T2 | 5508425 | 25-11-1993 |
| | | | JP B2 | 2816348 | 27-10-1998 |
| | | | KR B1 | 9710058 | 20-06-1997 |
| | | | LT A | 962 | 27-03-1995 |
| | | | LV A | 10584 | 20-04-1995 |
| | | | LV B | 10584 | 20-06-1996 |
| | | | US A | 5409771 | 25-04-1995 |
| | | | WO A1 | 9200108 | 09-01-1992 |
| EP A2 | 668080 | 23-08-1995 | JP A2 | 7310021 | 28-11-1995 |
| | | | US A | 5610208 | 11-03-1997 |
| | | | JP A2 | 7278225 | 24-10-1995 |
| EP A1 | 712659 | 22-05-1996 | EP A4 | 712659 | 27-12-1996 |
| | | | JP A2 | 8057310 | 05-03-1996 |
| | | | US A | 5760080 | 02-06-1998 |
| | | | WO A1 | 9534377 | 21-12-1995 |
| | | | JP A2 | 8057311 | 05-03-1996 |
| JP A2 | 8027372 | 30-01-1996 | none | | |

For more details about this annex see Official Journal of the European Patent Office, No. 12/82.